# EUROPÄISCHE PATENTANMELDUNG

(11) **EP 1 897 510 A1**
(43) Veröffentlichungstag der Anmeldung: **12.03.2008**
(21) Anmeldenummer: 07015331.7
(22) Anmeldetag: 04.08.2007
(51) Int. Cl.: A61B 18/14

(54) **Endoskopisches Hochfrequenzinstrument**

(30) Priorität: 07.09.2006 DE 102006041919
(71) Anmelder: OLYMPUS WINTER & IBE GMBH, 22045 Hamburg (DE)
(72) Erfinder: Wosnitza, Thomas, 21337 Lüneburg (DE)
(74) Vertreter: Hausfeld, Norbert

(57) **Zusammenfassung**

Ein Endoskopisches Hochfrequenzinstrument (1) mit einem einen durchgängigen Arbeitskanal (10) umschließenden Metallschaft (3) und einer im Arbeitskanal (10) längsverschiebbar gelagerten Elektrode (13), die über einen den Arbeitskanal (10) durchlaufenden Leiter (14) an einen von zwei Polen eines Hochfrequenzgenerators (18) angeschlossen ist, ist dadurch gekennzeichnet, daß der Metallschaft (3) an den anderen Pol des Generators (18) angeschlossen ist und daß der distale Endbereich des Arbeitskanals (10) mit einem Isolatorrohr (19) ausgekleidet ist, das das distale Ende des Arbeitskanals (10) um ein Stück überragt, das dem elektrischen Sicherheitsabstand (20) zwischen den Polen in leitfähiger Flüssigkeit entspricht.

## Beschreibung

Die Erfindung betrifft ein Hochfrequenzinstrument der im Oberbegriff des Anspruches 1 genannten Art.

Solche Hochfrequenzinstrumente dienen der chirurgischen Bearbeitung von Körpergewebe mittels Stromfluß von der Elektrode. Es können damit z.B. Tumore in der Blasenwand entfernt werden. Durch Verschieben der Elektrode im Arbeitskanal läßt sich der Operationsort erreichen. Der Metallschaft kann selbst als einfaches Rohr den Arbeitskanal ausbilden. Der Arbeitskanal kann jedoch auch in einem von dem Metallschaft umschlossenen Instrument, wie z.B. einem Zystoskop, ausgebildet sein, bei dem der Metallschaft nicht nur den Arbeitskanal, sondern auch eine Optik und einen Lichtleiter aufnimmt, so daß der Arbeitsort während der Arbeit unter Beleuchtung beobachtet werden kann.

Bekannte gattungsgemäße Hochfrequenzinstrumente sind mit einer an den zweiten Pol des Hochfrequenzgenerators angeschlossenen Neutralelektrode ausgerüstet, die von außen an den Körper des Patienten angelegt wird, so daß der Hochfrequenzstrom von der Elektrode durch das Körpergewebe zur Neutralelektrode fließt. Der flüssigkeitsgefüllte Raum, in dem die Elektrode liegt, z.B. die menschliche Blase, ist dabei mit nur schwach leitfähiger Flüssigkeit gefüllt.

Nicht gattungsgemäße Hochfrequenzinstrumente gemäß WO 97/00647 verwenden eine bipolare Elektrode, an der im Abstand und isoliert zueinander zwei Elektroden vorgesehen sind, die an die beiden Pole des Generators angeschlossen sind. Damit wird in hochleitfähiger, mit Salzlösung angereicherter Flüssigkeit gearbeitet, so daß der Strom unmittelbar zwischen den Elektroden durch die Flüssigkeit fließt. Damit wird der schädliche Stromdurchgang durch den Körper vermieden. Es ergibt sich jedoch eine sehr aufwendige Konstruktion.

Die Aufgabe der vorliegenden Erfindung ist es, ein Hochfrequenzinstrument zu schaffen, das bei geringem apparativem Aufwand bipolares Arbeiten in leitfähiger Flüssigkeit ermöglicht.

Diese Aufgabe wird mit den Merkmalen des Anspruches 1 gelöst.

Erfindungsgemäß kann eine einfache handelsübliche monopolare Nadelelektrode verwendet werden, die z.B. in üblicher Bauweise aus einem Leiterdraht besteht, der über die Länge der Elektrode isoliert ist und im Spitzenbereich an der eigentlichen Elektrode freiliegt. Diese Elektrode wird in einem einfachen Metallrohr oder z.B. in einem Zystoskop mit Arbeitskanal eingesetzt, wobei der Metallschaft an den zweiten Pol des Hochfrequenzgenerators angeschlossen ist. Der Strom fließt somit zwischen der Elektrode und dem Schaft auf kurzem Wege durch die leitfähige Flüssigkeit. Dabei ergibt sich jedoch ein Problem mit der elektrischen Sicherheit, da beim Längsverschieben der Elektrode diese in den Bereich der distalen Mündung des Arbeitskanals gelangen kann, wo sie in wenigen Zehntelmillimetern Abstand zu dem Metallschaft des Instrumentes liegt, der an den zweiten Pol angeschlossen ist. Es kann dann in stark leitfähiger Flüssigkeit ein so starker Lichtbogen entstehen, daß es zum Kurzschluß und sogar zum Verschweißen der Elektrode mit dem Schaft kommt. Die Erfindung hilft dem mit dem Isolatorrohr ab, das im distalen Endbereich des arbeotskanal, diesen distal überragend, angeordnet ist, wobei das Isolatorrohr den Arbeitskanal um ein solches Stück überragt, daß beim Zurückziehen der Elektrode der minimale Abstand zwischen Elektrode und Schaft eingehalten wird, der einen Kurzschluß verhindert.

Es reicht aus, wenn das Isolatorrohr nur im distalen Endbereich des Arbeitsrohres ausgebildet ist, um im Arbeitsbereich der Elektrode, in dem diese bei eingeschaltetem Strom vor- und zurückgeschoben wird, Kurzschluß zu verhindern. Vorteilhaft ist jedoch gemäß Anspruch 2 das Isolatorrohr über die gesamte Länge des Arbeitskanals erstreckt, was eine einfachere Montage ermöglicht und es au-ßerdem ermöglicht, daß die Elektrode auf ganzer Länge nicht isoliert ausgebildet sein kann.

Das Isolatorrohr kann z.B. als Keramikrohr ausgebildet sein, ist vorteilhaft jedoch gemäß Anspruch 3 in einfacher und kostengünstiger Weise als geeignet isolierender Kunststoffschlauch ausgebildet.

In der Zeichnung ist die Erfindung beispielsweise und schematisch dargestellt. Es zeigen:
- Fig. 1: eine Seitenansicht eines erfindungsgemäß ausgebildeten Zystoskopes,
- Fig. 2: einen Schnitt nach Linie 2-2 in Fig. 1 und
- Fig. 3: einen Schnitt nach Linie 3-3 in Fig. 2.

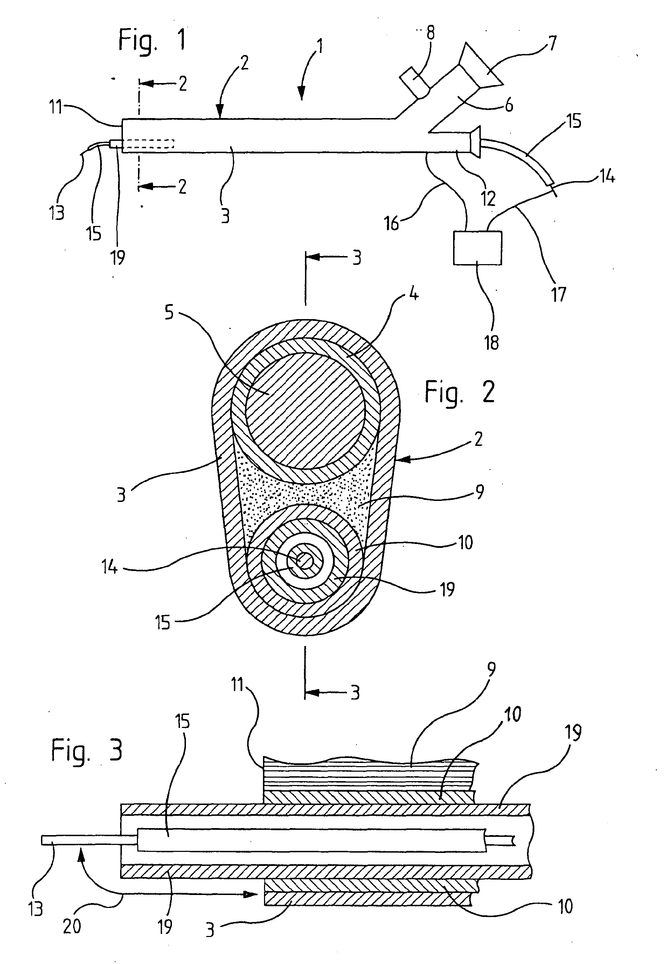

Die Fig. 1-3 zeigen ein endoskopisches Hochfrequenzinstrument 1 mit einem Schaft 2, der in Fig. 2 im Schnitt dargestellt ist. Ein metallenes Schaftrohr 3 umschließt den Schaft. In seinem Inneren ist in einem Optikrohr 4 eine Optik 5 angeordnet. Es kann sich dabei um eine Glasfaseroptik oder eine Linsenoptik handeln, die am distalen Ende ein Objektiv aufweist und in einem abgewinkelten proximalen Endstück 6 zu einem Okular 7 führt. Auch eine Videooptik ist verwendbar. Am Endstück 6 sitzt ferner ein Lichtleiteranschlußstutzen 8, von dem aus ein Glasfaserlichtleiter 9 die freien Querschnittsteile im Schaftrohr 3 bis zu dessen Stirnfläche durchläuft.

Im Inneren des Schaftrohres 9 ist ferner ist ein von einem Kanalrohr 10 gebildeter Arbeitskanal vorgesehen, der am distalen Ende in der Stirnfläche 11 des Schaftes 2 mündet und am proximalen Ende an einem Eingangsstutzen 12 mündet.

Durch den im Kanalrohr 10 ausgebildeten Arbeitskanal ist eine Nadelelektrode 13 verlegt, die als freies Ende eines Leiterdrahtes 14 ausgebildet ist, der bis auf den freiliegenden Spitzenbereich 13 mit einer Isolierung 15 versehen ist. Das Schaftrohr 3 und der Leiterdraht 14 der Nadelelektrode 13 sind über Leitungen 16 und 17 an die beiden Pole eines Hochfrequenzgenerators 18 angeschlossen.

Das in den Figuren dargestellte Instrument 1 wird z.B. in der menschlichen Blase eingesetzt, um mit der Nadelelektrode 13 Gewebe in der Blasenwand, z.B. einen kleinen Tumor, durch Hochfrequenzstromeinwirkung zu beseitigen. Bevorzugt wird dabei der Raum um die Nadelelektrode 13 mit gut leitfähiger, salzhaltiger Flüssigkeit versorgt. Der Strom fließt dabei von der Nadelelektrode 13 zum Schaftrohr 3. Es entsteht dabei bevorzugt ein Plasma um die Nadelelektrode 13, mit dem Gewebe abgetragen wird.

Die Nadelelektrode 13 ist im Arbeitskanal 10 axial verschiebbar und kann während des Stromflusses axial bewegt werden, um entsprechend benötigte Arbeitsbewegungen auszuführen. Gelangt dabei die Nadelektrode 13 in die Nähe der Stirnfläche 11 des Schaftrohres 3 oder des mit diesem elektrisch verbundenen, ebenfalls aus Metall bestehenden Kanalrohres 10, so kann der Strom zu hoch werden und den Hochfrequenzgenerator 18 überlasten. Es kann sogar zum Verschweißen kommen.

Um aufwendige Sicherungsmaßnahmen zu vermeiden, ist ein Isolatorrohr 19 vorgesehen, das, wie in der Detailansicht der Fig. 3 am besten ersichtlich, im Kanalrohr 10 angeordnet ist und dessen Stirnfläche 11 um ein Stück überragt. Wird, wie Fig. 3 zeigt, die eingeschaltete Nadelelektrode 13 zu weit zurückgezogen, besteht also Gefahr, daß sie zu nahe an den anderen elektrischen Pol am Schaftrohr 3 gelangt, so wird sie von dem die Stirnfläche 11 überragenden Stück des Isolatorrohres 19 abgeschirmt, so daß zwischen der Nadelelektrode 13 und der Stirnfläche 11 ein elektrischer Mindestabstand 20 eingehalten wird, bei dem elektrische Überlastung noch nicht eintritt. Weitere elektrische Schutzmaßnahmen können mit diesem einfachen Hilfsmittel eingespart werden.

Das Isolatorrohr 19 kann, wie Fig. 1 gestrichelt zeigt, als kurzes Rohrstück im distalen Endbereich des Schaftes 3 angeordnet sein, kann jedoch auch über die gesamte Länge des Kanalrohres 10 ausgebildet sein. Dadurch wird die Montage des Isolatorrohres 19 erleichtert und es ergibt sich die Möglichkeit, die Nadelelektrode 13 auch ohne die Isolierung 15 zu verwenden, wenn diese nicht aus anderen Gründen, z.B. der Konzentrierung des Stromflusses auf die freiliegende Länge der Nadelelektrode 13, benötigt wird.

Das Isolatorrohr 19 kann aus geeignet isolierendem Material, wie Keramik, bestehen oder als Kunststoffschlauch ausgebildet sein. Es kann im Kanalrohr 10 auf geeignete Weise, z.B. durch Verklebung, befestigt sein.

In einer nicht dargestellten stark vereinfachten Ausführungsvariante kann das Hochfrequenzinstrument lediglich aus dem Kanalrohr 10 bestehen, das mit der Leitung 16 an den Hochfrequenzgenerator 18 angeschlossen ist. Die Elektrodenanordnung 13, 15, 14 und das Isolatorrohr 19 sind in der zuvor beschriebenen Weise im Kanalrohr 10 angeordnet. Mit einem solchen Instrument muß allerdings ohne optische Beobachtung gearbeitet werden.

## Patentansprüche

1. Endoskopisches Hochfrequenzinstrument (1) mit einem einen durchgängigen Arbeitskanal (10) umschließenden Metallschaft (3) und einer im Arbeitskanal (10) längsverschiebbar gelagerten Elektrode (13), die über einen den Arbeitskanal (10) durchlaufenden Leiter (14) an einen von zwei Polen eines Hochfrequenzgenerators (18) angeschlossen ist, **dadurch gekennzeichnet, daß** der Metallschaft (3) an den anderen Pol des Generators (18) angeschlossen ist und daß der distale Endbereich des Arbeitskanals (10) mit einem Isolatorrohr (19) ausgekleidet ist, das das distale Ende des Arbeitskanals (10) um ein Stück überragt, das dem elektrischen Sicherheitsabstand (20) zwischen den Polen in leitfähiger Flüssigkeit entspricht.

2. Instrument nach Anspruch 1, **dadurch gekennzeichnet, daß** das Isolatorrohr (19) sich über die gesamte Länge des Arbeitskanals (10) erstreckt.

3. Instrument nach Anspruch 1, **dadurch gekennzeichnet, daß** das Isolatorrohr (19) als Kunststoffschlauch ausgebildet ist.
